(19) Europäisches Patentamt
European Patent Office
Office européen des brevets

(11) Publication number: **0 248 259 B1**

(12) # EUROPEAN PATENT SPECIFICATION

(45) Date of publication of patent specification: **17.04.91 Bulletin 91/16**

(51) Int. Cl.[5]: **C07C 53/02,** C07C 51/06, C07C 259/06

(21) Application number: **87107197.3**

(22) Date of filing: **18.05.87**

(54) **Process for producing formic acid by carbonylation of hydroxyalkylamines and hydrolysis of the hydroxyalkylformamides produced.**

(30) Priority: **20.05.86 IT 2048486**

(43) Date of publication of application: **09.12.87 Bulletin 87/50**

(45) Publication of the grant of the patent: **17.04.91 Bulletin 91/16**

(84) Designated Contracting States: **AT BE CH DE ES FR GB GR IT LI LU NL SE**

(56) References cited: **US-A- 4 510 326**

(73) Proprietor: **CONSIGLIO NAZIONALE DELLE RICERCHE**
**Piazzale Aldo Moro, 7**
**I-00198 Roma (IT)**

(72) Inventor: **Ricci, Alessandro**
**Via Sirio Moggi 131**
**Visignano/Cascina Pisa (IT)**
Inventor: **Sbrana, Glauco**
**Via Francesco Rismondo 31**
**Pisa (IT)**
Inventor: **Braca, Giuseppe**
**V.le delle Piagge 8**
**Pisa (IT)**

(74) Representative: **Gervasi, Gemma, Dr. et al**
**NOTARBARTOLO & GERVASI Srl 33, Viale Bianca Maria**
**I-20122 Milano (IT)**

EP 0 248 259 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a process for producing formic acid by carbonylation of hydroxyalkylamines and hydrolysis of the hydrohyalkylformamides produced.

More particularly, the invention relates to a process for producing formic acid by carbonylation of hydroxyalkylamines in the absence of a catalyst, and hydrolysis of the resultant hydroxyalkylformamides in the presence of acid catalysts.

Said process is conveniently applicable on an industrial scale because of its simplicity and high yields.

Formic acid preparation processes starting from carbonylation of methanol are known, and are described for example in Hydroc. Proc 59, (11), 120 (1980) and in Oil and Gas Journal Nov. 16, 1981. According to one of these processes, the formic acid is prepared by carbonylation of methanol, ammonolysis of the methyl formate produced, and hydrolysis of the formamide in accordance with the following reactions :

$$CO + CH_3OH \rightleftarrows HCOOCH_3 \quad (I)$$
$$HCOOCH_3 + NH_3 \rightarrow HCONH_2 + CH_3OH \quad (II)$$
$$2HCONH_2 + H_2SO_4 + 2H_2O \rightarrow 2HCOOH + (NH_4)_2SO_4 \quad (III)$$

Reaction (I) is an equilibrium reaction which is not very favourable in terms of formate formation under industrially convenient conditions.

The reason for this is that if operating at low temperature, the reaction rate is low whereas if operating at high temperature the conversion is low.

Further drawbacks of this process are that reaction (I) requires the presence of an alkaline alcoholate catalyst, whereas reaction (III) leads to the formation of ammonium sulphate and thus involves sulphuric acid and ammonia consumption.

Finally, this is a three-stage process which on an industrial scale requires complicated plant, because of the need to separate the catalyst and ammonium sulphate.

According to another process described in the same publication, the formic acid is produced by direct hydrolysis of the methyl formate obtained by methanol carbonylation, in accordance with the reactions :

$$CO + CH_3OH \rightleftarrows HCOOCH_3 \quad (I)$$
$$HCOOCH_3 + H_2O \rightleftarrows HCOOH + CH_3OH \quad (IV)$$

With regard to reaction (I) the aforesaid comments are valid, whereas with regard to reaction (IV) this is a reaction which is very limited thermodynamically, so that it is necessary to operate with a very large excess of water to increase formate conversion, and this involves considerable cost in the recovery and recycling of the unconverted reagents.

In order to obviate this drawback a modification to this process has been proposed, consisting of conducting the hydrolysis reaction in the presence of 1-pentylimidazole which subtracts the formic acid from the equilibrium state by the formation of a salt from which the acid can be recovered by thermal decomposition and distillation.

However, apart from the high cost of 1-pentylimidazole, decomposition of the formed salt is difficult because of the hygroscopic nature of 1-pentylimidazole which becomes released in a water-rich environment.

This process is also a three-stage process, requiring complex plant for its industrial implementation because of the need to separate and recover the various products.

In both processes, for example, because of the presence of the alcoholates used as catalysts in the carbonylation reaction, the formate must be separated before subjecting it to ammonolysis or hydrolysis.

Processes involving carbonylation of primary and secondary amines and of hydroxyalkylamines are also known in the art.

In particular, the known processes for producing hydroxyalkylformamides by hydroxyalkylamine carbonylation involve the use of catalysts based on transition metals [Bull. Chem, Soc. Jpn., 42, 2610 (1969) and Uzb. Khim., 12. 37 (1968) ; C.A., 70, 46768 (1969)], or of iron-based catalysts [Izv. Akad. Nauk, SSSR Ser. khim., 2718 (1973) ; C.A., 80, 82589 n (1974)], or of Ni and Co metal carbonyl derivative : (Brit. Pat. 777,581 ; C.A., 51, 17983 i (1957)].

The need to conduct the hydroxyalkylamine carbonylation in the presence of said catalysts has meant that this process cannot be used for the industrial production of formic acid, and its application has been limited to the preparation of alkanolamides.

The preparation of certain hydroxyalkylformamides by hydroxyalkylamines carbonylation in the absence of catalyst is described in US-A-4510236.

It thus becomes possible to implement a process having the advantage of operating in the absence of often

labile substances extraneous to the reaction, which can give rise to the formation of by products and which, in all cases must be separated and recovered.

The formic acid production process according to the present invention is characterised by in a first stage reacting a primary or secondary hydroxyalkylamine directly with carbon monoxide and in a second stage treating the reaction mixture originating from the first stage with water and a strong acid catalyst and continuously separating the formic acid by decomposing the hydroxyalkylaminium formate produced. These and further characteristics and advantages of the process according to the present invention will be more apparent from the detailed description given hereinafter.

Said process is conducted in two stages :

– the first stage consists of direct carbonylation without catalyst of a primary or secondary hydroxyalkylamine with carbon monoxide to produce the corresponding hydroxyalkylformamides in accordance with the reactions :

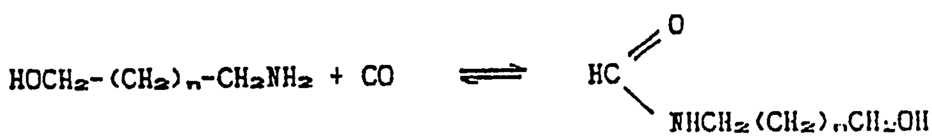 (V)

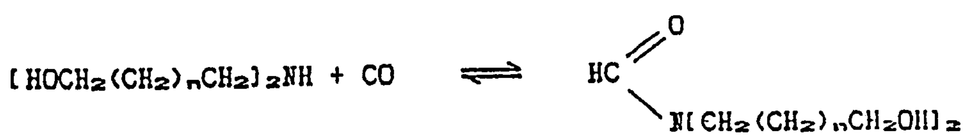 (VI)

in which n is a whole number between 0 and 10 ;

– the second stage consists of hydrolysing the hydroxyalkylformamides to obtain formic acid and regenerating the initial hydroxyalkylamines, in accordance with the reaction :

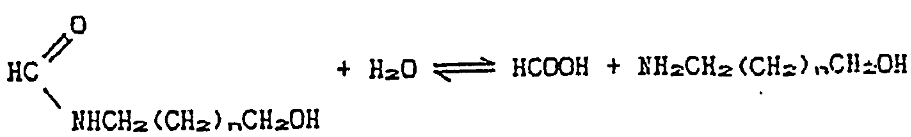 (VII)

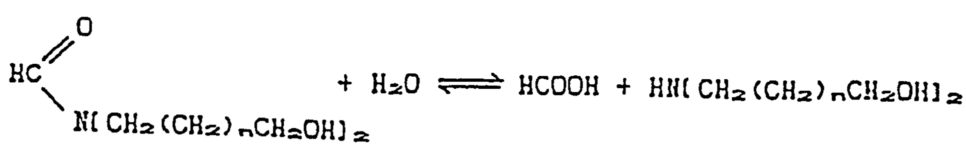 (VIII)

The formic acid which is released forms with the hydroxyalkylamine the corresponding thermally labile hydroxyalkylammoniun salt, from which the acid is released by stripping with steam as follow :

$$[NH_3CH_2(CH_2)_nCH_2OH]^+[HCOO]^- \rightleftharpoons HCOOH + NH_2CH_2(CH_2)_nCH_2OH \quad (IX)$$

The carbonylation reactions (V) and (VI) are conducted in autoclave at a temperature of between 70 and 250°C and preferably between 100 and 150°C at a pressure of between 1 and 200 bars and preferably between 50 and 120 bars.

When operating under the preferred conditions and using a primary hydroxyalhylamine as the carbonylation substrate, conversions into the corresponding formamide of up to 97% are obtained, with a productivity of 2-5 moles per litre of reactor per hour.

When operating under the same conditions with secondary hydroxyalkylamines, the carbonylation takes

place with equally high conversations, but with a lower reaction rate.

Hydroxyalkylamines which can be used in the present process are monoethanolamine, monopropanolamine, diethanolamine and higher mono and dialkanolamines.

The hydrosis reactions (VII) and (VIII) are conducted using a water/hydroxyalkylformamide molar ratio of between 20 : 1 and 1 : 1 and preferably between 10 : 1 and 2 : 1.

For these reactions the catalyst used us an acid chosen from the group comprising : formic acid, a strong but possibly little volatile organic acid such as oxalic acid, stong inorganic acids such as $H_2SO_4$ and HCl, inorganic acid oxides such as $Al_xO_4$ and zeolites, and acid ion exchange resins.

The use of formic acid as catalyst, even though it has an unfavourable effect on equilibrium, is definitely preferred as it avoids the introduction of foreign compounds into the system.

The catalyst concentration is between 0.3 and 0.7 N and preferably between 0.45 and 0.55 N. If formic acid is used, it increases with the progress of the reaction by virtue of its release by hydrolysis and decomposition of the hydroxyalkylammonium salt, and must therefore be continuously removed to maintain optimum concentration.

Removal of the formic acid formed by hydrolysis is effected by stripping with steam under pressure.

Strong inorganic acids, although functioning as hydrolysis catalysts, have the drawback of forming stable salts with the hydroxyalkylamine, thus becoming progressively deactivated.

Heterogeneous acid catalysts such as acid $Al_2O_3$ have the advantage of being easily separated from the liquid reaction mixture from which the formic acid is to be separated, and can be recycled.

The hydrolysis is conducted at a temperature of between 80 and 150°C. The higher temperatures (100-150°C) are preferred as they result in a higher reaction rate and more rapid removal of the formic acid from the reaction mixture, with favourable effect on the displacement of equilibrium.

From the aqueous solution obtained by stripping, formic acid is recovered in the pure state by means of known azeotropic distillation processes using diisopropylether or a formic ester as the third component.

The hydroxyalkylamine regenerated by the hydrolysis reaction, and possibly containing small quantities of the acid used as catalyst and unseparated formic acid, can be recycled to the carbonylation stage without the need to separate the acid components.

The process according to the invention is illustrated on the schematic diagram shown in Figure 1, and is much simpler and more advantageous than processes of the known art [Hydroc. Proc. 59, 11, 120-130 (1980)].

In said figure, the reference numerals 1 and 2 represent respectively the CO feed and recycle lines, 3 and 4 represent respectively the hydroxyalkylamine feed and recycle lines, and 5 represents the reactor in which the carbonylation is conducted ; 6 represents the line through which the mixture after carbonylation is transferred to the degasser 7 ; the degassed mixture after mixing with water and acid from the line 9 is transferred through the line 8 to the hydrolysis reactor 10 ; steam is fed into the bottom of said reactor through the line 11, and at the top of this reactor a mixture of vaporised formic acid and water develops which is transferred through the line 12 to condensation.

The following examples of the preparation of formic acid by the process of the present Invention are given hereinafter by way of non-limiting illustrution.

## EXAMPLE 1

1st stage : carbonylation of monoethanolamine.

3.3 moles of monoethanolamine and carbon monoxide at a pressure of 100 bars are fed into a reactor having a volume of 1000 ml. It is heated to 150°C and kept under these conditions for 2 hours, under agitation. A 60% conversion of monoethanolamine into hydroxyethylformamide is obtained, with a productivity of 5 moles of hydroxyethylformamide per litre of reactor per hour. On heating under the same conditions for a further 3 hours, a 93% conversion is obtained with an overall productivity of 3.1 moles per litre of reactor per hour. The reation mixture has a hydroxyethylformamide content of 95% by weight.

2nd stage : hydrolysis of hydroxyethylformamide.

3.1 moles of hydroxyethylformamide, 10 moles of water and 0.19 moles of HCOOH (acid concentration in solution in 0.5 N) as catalyst are fed into a reactor having a volume of 1000 ml.

The mixture is heated to 140°C and this temperature maintained for 3 hours. The conversation of hydroxyethylformamide is 22%. 0.40 moles of formic acid are recovered by distilling the reaction mixture in a current of steam under pressure at 150°C. After formic acid recovery, the solution was again subjected to carbonylation with CO at 100 bars at a temperature of 150°C. The reaction proceeded until complete carbonylation of the free ethanolamine, with the formation of hydroxyethylformamide, which reaches a concentration of 80% in the solution.

EXAMPLES 2-4

Carbonylation of hydroxyalkylamines to hydroxyalkylformamides. Monoethanolamine, diethanolamine and monopropanolamine were treated with carbon monoxide under various temperature, pressure and time conditions.

The conversion at various reaction times was particularly determined in these examples.

The operating conditions and results obtained are given in Table 1.

TABLE 1 - Examples of carbonylation of hydroxyalkylamines to hydroxyalkylformamides

| Example No. | Substrate (moles) | Temp. °C | Pressure bars | Time h | Conversion % | Hydroxyalkyl-formamide % by weight | Productivity (*) |
|---|---|---|---|---|---|---|---|
| 2 | monoethanol-amine (3,38) | 100 | 90 | 3 | 18,3 | 24,6 | 1,1 |
| | | 100 | 90 | 9 | 50,5 | 59,9 | 0,6 |
| | | 100 | 90 | 9 | 75,2 | 81,6 | 0,46 |
| | | 150 | 90 | 3 | 97,4 | 98,5 | 1,24 |
| 3 | diethanol-amine (0,26) | 150 | 120 | 8 | 75,0 | 79,2 | 0,97 |
| | | 150 | 120 | 8 | 92,9 | 94,4 | 0,23 |
| 4 | monopropanol-amine (0,55) | 100 | 90 | 8 | 1,0 | 1,3 | 0,014 |
| | | 150 | 90 | 16 | 77,8 | 82,8 | 0,66 |

(*) Moles of hydroxyalkylformamide produced per litre of reactor per hour.

EXAMPLES 5-14

Hydrolysis of hydroxyalkylformamide to formic acid and hydroxyalhylamine.

In these examples the hydroxyalkylformamide is hydrolysed with water in the presence of an acid catalyst, while varying the water/substrate ratio and catalyst/substrate ratio, the type of catalyst, the temperature and the time.

The operating conditions and the results are given in Table 2.

TABLE 2 - Examples of hydrolysis of hydroxyalkylformamide to formic acid and hydroxyalkylamine

| Example No. | Reagents hydroxyethyl-formamide mmoles | water mmoles | Catalyst type | mmoles | Normality | Temp. °C | Time h | Conversion % |
|---|---|---|---|---|---|---|---|---|
| 5 | 275 | 841 | HCOOH | 18 | 0,5 | 100 | 13 | 22,0 |
| 6 | 217 | 2200 | HCOOH | 32 | 0,5 | 150 | 8 | 42,0 |
| 7 | 495 | 2628 | $(COOH)_2$ | 20 | 0,23 | 100 | 20 | 45,0 |
| 8 | 381 | 222 | $H_2SO_4$ | 15 | 1,0 | 100 | 4,5 | 29,0 |
| 9 | 381 | 777 | $H_2SO_4$ | 15 | 1,0 | 70 | 10 | 31,0 |
| 10 | 152 | 791 | Amberlyst 3,8 g | 15 | 0,5 | 100 | 15 | 25 |
| 11 | 233 | 717 | Zeolite HZSM5 1,5 g | - | | 100 | 19 | 25,0 |
| 12 | 247 | 909 | $Al_2O_3$ 504C Fluka 2,65 g | - | | 100 | 10 | 28,4 |
| 13 | 310 | 1580 | Idem 3,3 g | | | 150 | 4 | 33 |
| | N,N-dihydroxyethyl-formamide | | | | | | | |
| 14 | 1910 | 5730 | HCOOH | 141 | 0,5 | 140 | 6 | 8,5 |

## Claims

1. A process for the production of formic acid by carbonylation of hydroxyalkylamines to hydroxyalkylformamides and hydrolysis of these latter to formic acid characterised in that

– in a first stage a primary or secondary hydroxyalkylamine is reacted with carbon monoxide, in the absence of catalyst, at a temperature of between 70° and 250°C and at a pressure of between 1 and 200 bars ;

– in a second stage the hydroxyalkylformamide-containing reaction mixture is treated with water, using a water : hydroxyalylformamide molar ratio of between 20 : 1 and 1 : 1, and a strong acid catalyst, at a temperature of between 80° and 150°C and the obtained formic acid is continously separated by stripping with steam.

2. A process as claimed in claim 1, characterised in that said first stage reaction is conducted at a temperature of between 100° and 150°C and at a pressure of between 50 and 120 bars.

3. A process as claimed in claim 1, characterised in that said second stage reaction is conducted using a water : hydroxyalylformamide molar ratio of between 10 : 1 and 2 : 1.

4. A process as claimed in claim 1, characterised in that said strong inorganic acid is selected from the group comprising formic acid, oxalic acid, sulfuric acid, hydrochloric acid, alumina, zeolites and acid ion exchange resins.

5. A process as claimed in claim 1, characterised in that when said strong acid catalyst is soluble, its concentration is of between 0.3 and 0.7 N.

6. A process as claimed in claim 5, characterised in that said strong acid catalyst concentration is of between 0.45 and 0.55 N.

## Ansprüche

1. Verfahren zur Herstellung von Ameisensäure durch Carbonylierung von Hydroxyalkylaminen zu Hydroxyalkylformamiden und Hydrolyse dieser letzteren zu Ameisensäure, dadurch gekennzeichnet, daß

- in einer ersten Stufe ein primäres oder sekundäres Hydroxyalkylamin mit Kohlenmonoxid in Abwesenheit eines Katalysators bei einer Temperatur von 70 bis 250°C und bei einem Druck von 1 bis 200 bar umgesetzt wird,
- in einer zweiten Stufe die hydroxyalkylformamidhaltige Reaktionsmischung mit Wasser unter Anwendung eines Molverhältnisses Wasser : Hydroxyalkylformamid von 20 : 1 bis 1 : 1 und einem stark sauren Katalysator bei einer Temperatur von 80 bis 150°C behandelt und die erhaltene Ameisensäure durch Abziehen mit Dampf kontinuierlich abgetrennt wird.

2. Verfahren nach Anspruch 1, dadurch gekennzeichnet, daß die erste Reaktionsstufe bei einer Temperatur von 100 bis 150°C und bei einem Druck von 50 bis 120 bar durchgeführt wird.

3. Verfahren nach Anspruch 1, dadurch gekennzeichnet, daß die zweite Reaktionsstufe unter Anwendung eines Molverhältnisses Wasser : Hydroxyalkylformamid von 10 : 1 bis 2 : 1 durchgeführt wird.

4. Verfahren nach Anspruch 1, dadurch gekennzeichnet, daß die starke anorganische Saure ausgewählt wird aus der Gruppe umfassend Ameisensäure, Oxalsaure, Schwefelsäure, Salzsäure, Aluminiumoxid, Zeolithe und saure Ionenaustauscherharze.

5. Verfahren nach Anspruch 1, dadurch gekennzeichnet, daß, wenn der stark saure Katalysator löslich ist, seine Konzentration 0,3 bis 0,7 n beträgt.

6. Verfahren nach Anspruch 5, dadurch gekennzeichnet, daß die Konzentration des stark sauren Katalysators 0,45 bis 0,55 n betragt.

## Revendications

1. Procédé de fabrication d'acide formique par carbonylation d'hydroxyalkylamines en hydroxyalkylformamides et hydrolyse de ces derniers en acide formique, caractérisé en ce que :

- dans une première étape, on fait réagir une hydroxyalkylamine primaire ou secondaire avec le monoxyde de carbone, en l'absence de catalyseur, à une température se situant entre 70°C et 250°C et sous une pression se situant entre 1 et 200 bars ;
- dans une seconde étape, on traite le mélange réactionnel contenant l'hydroxyalkylformamide avec de l'eau, en utilisant un rapport moléculaire eau/hydroxyalkylformamide se situant entre 20/1 et 1/1 et en utilisant un catalyseur à base d'acide fort, à une température se situant entre 80°C et 150°C, et l'acide formique obtenu est séparé en continu par rectification à la vapeur d'eau.

2. Procédé tel que revendiqué dans la revendication 1, caractérisé par le fait que la réaction au cours de la première étape est effectuée à une température se situant entre 100°C et 150°C et sous une pression se situant entre 50 et 120 bars.

3. Procédé tel que revendiqué dans la revendication 1, caractérisé par le fait que la réaction au cours de la seconde étape est effectuée en utilisant un rapport moléculaire eau/hydroxyalkylformamide se situant entre 10/1 et 2/1.

4. Procédé tel que revendiqué dans la revendication 1, caractérisé par le fait que l'acide fort minéral est choisi parmi le groupe constitué par l'acide formique, l'acide oxalique, l'acide sulfurique, l'acide chlorhydrique, l'alumine, des zéolites et des résines échangeuses d'ions acides.

5. Procédé tel que revendiqué dans la revendication 1, caractérisé par le fait que lorsque le catalyseur à base d'acide fort est soluble, sa concentration se situe entre 0,3 N et 0,7 N.

6. Procédé tel que revendiqué dans la revendication 5, caractérisé par le fait que la concentration du catalyseur à base d'acide fort se situe entre 0,45 N et 0,55 N.

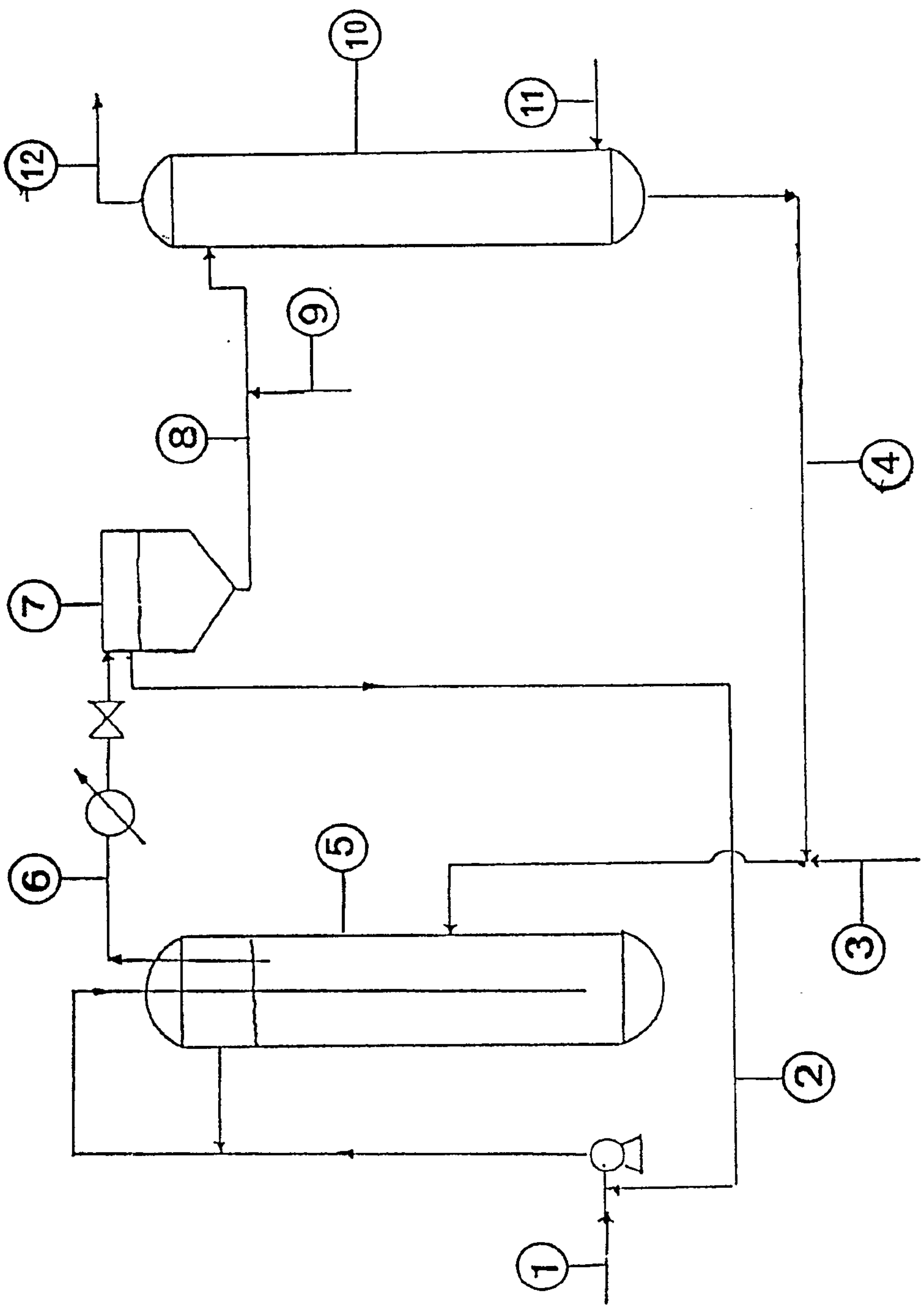


FIG.1